# EUROPEAN PATENT APPLICATION

(11) **EP 1 174 416 A1**
(43) Date of publication of application: **23.01.2002**
(21) Application number: 00917463.2
(22) Date of filing: 25.04.2000
(51) Int. Cl.: C07C 69/587, A23D 9/00, A61K 31/201, A61K 9/48, A61K 9/20, A61P 3/04, A61P 3/06, A23L 1/30

(54) **CONJUGATED FATTY ACID ESTERS**

(30) Priority: 27.04.1999 JP 12070699
(71) Applicant: Kabushiki Kaisha Yakult Honsha, Minato-ku, Tokyo 105-8660 (JP)
(72) Inventor: KUDO, Satoshi, Kabushiki Kaisha Yakult Honsha, Tokyo 105-8660 (JP); MIZUSAWA, Naomi, Kabushiki Kaisha Yakult Honsha, Tokyo 105-8660 (JP); HAMURA, Mahoko, Kabushiki Kaisha Yakult Honsha, Tokyo 105-8660 (JP)
(74) Representative: Warcoin, Jacques
(86) International application number: JP0002703
(87) International publication number: WO0064854

(57) **Abstract**

Use of conjugated fatty acid glycerides, which are formed by converting conjugated fatty acids having conjugated double bond(s) in the molecule into glycerol esters, to more effectively exert the inherent physiological effects of the conjugated fatty acids and control the bitterness or astringency of the conjugated fatty acids, thereby making the conjugated fatty acids suitable for oral intake. These conjugated glycerides (for example, glycerides having conjugated linoleic acid in the molecule) have effects of improving lipid metabolism, preventing obesity, and preventing and treating hypertension.

## Description

### FIELD OF THE INVENTION

The present invention relates to a conjugated fatty add ester structure for effective incorporation of a conjugated fatty acid with conjugated double bonds within the molecule, particularly to the mono-, di- or triglyceride of the conjugated fatty acid in the form of glycerol ester. More specifically, the invention relates to a conjugated fatty acid glyceride having conjugated linoleic acid with an action to improve lipid metabolism, an action to reduce neutral fat as a risk factor of arteriosclerosis and cardiovascular diseases and an action to improve hyperlipidemia, within the molecule. The glyceride can be used in medical products and foods and drinks.

### BACKGROUND OF THE INVENTION

Following the change of the life environment in recent years, the increase of diseases derived from daily life habits (obesity, diabetic mellitus, hyperlipidemia and the like) and caused by excess food intake and insufficient sport activities, has been remarked. The diseases now increasingly occur in lower ages. Among them, hyperlipidemia as one of lipid metabolism abnormalities has a close relation with the onset of arteriosclerosis. Thus, importantly, blood lipid level should be controlled. These diseased conditions can fairly be improved by exercise therapy and diet therapy. However, both the therapies have required considerable efforts for the execution and instructions from doctors and dietitians.

Alternatively, conjugated fatty acids in nature, which can also be generated during food production process, are fatty acids where adjacent carbons with a single bond interposed therein have double bonds. Conjugated linoleic acid with one conjugated diene within the fatty acid molecule with 18 carbon atoms, or eleostearic acid with two conjugated dienes or parinaric acid with three conjugated dienes has been known well. The physiological activities thereof have also been researched.

As to conjugated linoleic acid, in particular, many useful physiological activities thereof have been reported in recent years. For example, JP-A-8-505775, corresponding to International Publication No. WO 94/16690, discloses a method for enhancing weight gain and feed efficiency in an animal, which comprises administering to the animal a conjugated linoleic acid. Also, JP-A-10-508189, corresponding to International Publication No. WO 96/06605, discloses a method for reducing body fat in an animal, which comprises administering to the animal a conjugated linoleic acid. Additionally, reports have been issued about the anti-cancer effect, anti-allergy effect and the like, other than those described above.

It is known that conjugated linoleic acid is a fatty acid existing in the form of glyceride in plants; and that conjugated linoleic acid is converted and generated from linoleic acid with some microorganisms in the digestive tracts of animals, for absorption.

Industrial production and marketing of conjugated linoleic acid with such various physiological actions as described above has been started. Commercially available conjugated linoleic acid products include for example CLA 80 Active Linol, manufactured by RINORU OIL MILLS CO., LTD. and Tonalin (Trademark) of Maypro Co., Ltd. These are produced by conjugating cis-type linoleic acid (C 18 : 2) of natural type in the presence of alkali, and these contain cis-9, trans-11 or trans-9, cis-11, trans-10, cis-12 free fatty acids as the principal components as well as some isomers positional or geometric.

However, products of these free fatty acid types have such strong bitterness and astringency that the products frequently cause difficulty in the incorporation thereof as they are or in the addition thereof at their physiologically effective amounts to general foods.

Further, conjugated linoleic acids are commercially available in the form of dietary supplements of capsule or tablet types in many cases. When these formulations are solely incorporated, particularly between meals, conjugated linoleic acids are poor in terms of digestion and absorption, disadvantageously.

Meanwhile, attempts have been made to extract and utilize a vast amount of glyceride-type conjugated fatty acids from plants and the like. However, not any industrially cost-effective raw material therefor has been known.

Hence, it has been attempted to synthetically prepare conjugated fatty acid glyceride via enzymatic reaction. For example, some attempts about the modification of conjugated linoleic acid into glyceride derivatives by general processes such as ester exchange reaction process or ester synthesis process have been reported (Garcia, H.S. et al., Biotechnology Letters, Vol. 20, No. 4, 1998, pp.393-395; Arcos, J.A. et al., Biotechnology Letters, Vol. 20, No. 6, 1998, pp. 617-621).

However, these reports never include any description about the sensory qualities and physiological effects of the generated glyceride derivatives, although these describe the possibility of producing conjugated linoleic acid in the mono-, di- and tri-glyceride derivatives by utilizing synthetic reaction with lipase.

### SUMMARY OF THE INVENTION

The present invention provides the use of conjugated fatty acid ester for oral intake of conjugated fatty acids typically including conjugated linoleic acid, after the bitterness and astringency of conjugated fatty acids are suppressed in order to produce agents for improving lipid metabolism, anti-obesity agents, and prophylactic and therapeutic agents of hyperlipidemia. More specifically, in a method of preparing conjugated fatty acids suitable for oral intake so as to allow the conjugated fatty acids to more effectively exert the essential physiological effects thereof and to suppress the bitterness and astringency of the conjugated fatty acids, in accordance with the present invention, use is made of a conjugated fatty acid glyceride prepared by modifying a conjugated fatty acid with conjugated double bonds within the molecule into the form of glycerol ester.

The conjugated fatty acid glyceride according to the present invention has no bitterness or astringency, in spite of any mode of oral intake, so the conjugated fatty acid glyceride can efficiently be absorbed.

In accordance with the present invention, specifically, the conjugated fatty acid glyceride of a conjugated fatty acid in the form of glycerol ester, among conjugated fatty acid esters with conjugated fatty acids within the molecules, can be used as an agent for improving lipid metabolism, an anti-obesity agent, and an agent for preventing and therapeutically treating hyperlipidemia.

Additionally, the present invention provides glycerides containing conjugated linoleic acid glyceride and having high safety profiles, which can be ingested daily as food.

In a preferred embodiment, the present invention provides a conjugated fatty acid glyceride with a conjugated linoleic acid within the molecule, as the conjugated fatty acid with conjugated double bonds.

In another preferred embodiment, the present invention provides a conjugated fatty add glyceride having 9,11-octadecadienoic acid or 10,12-octadecadienoic acid as the conjugated fatty acid with conjugated double bonds.

Additionally, in a still another preferred embodiment, the present invention provides a conjugated fatty acid glyceride in any one form selected from the group consisting of mono-, di- or triglycerides.

The conjugated fatty acid glyceride according to the present invention can be supplied in foods and drinks, by mixing the conjugated fatty acid glyceride with foods and drinks causing no modification of the glyceride, such as milk and soybean milk, and can also be supplied in medical products by blending therein other pharmaceutical substances or adjuvants causing no modification of the glyceride, according to general methods for capsules and tablets.

As the method for producing the conjugated fatty acid glyceride of the present invention, use may be made of any of a method comprising conjugation of cis-type linoleic acid of natural type in the presence of alkalis and subsequent ester synthesic reaction or ester exchange reaction using catalysts or a method comprising conjugation of fatty acid-containing glyceride.

Among them, ester exchange or ester synthetic reaction using a biological catalyst lipase as the catalyst is preferable for use in foods. Particularly, ester synthetic reaction is preferable. Ester synthetic reaction may satisfactorily be progressed by general methods. For example, conjugated linoleic acid may satisfactorily react under dehydration in the presence of immobilized lipase and glycerol. According to the method, the content of conjugated fatty acid in total fat can prominently be increased. Additionally, the reaction can progress at ambient temperature, and the inactivation and removal of the catalyst can readily be done, while limiting side reactions such as fat oxidation so that the method is highly safe.

Although ester exchange reaction is highly safe, the reaction yield is poor, while it is difficult to separate a byproduct fatty acid. Although glyceride can be synthetically prepared with chemical catalysts, alternatively, unexpected byproducts may emerge or the resulting glyceride yield may be insufficient. Additionally, it is laborious to remove the chemical catalysts.

The oils and fats as the substrates of ester exchange reaction include vegetable fats and oils, such as soybean oil, corn oil, cottonseed oil, sunflower oil, safflower oil, rapeseed oil, olive oil, peanut oil, linseed oil, perilla oil, coconut oil and palm oil. The animal fats and oils may also be used as the substrates of ester exchange reaction, and these animal fats and oils include fish oils, such as sardine oil, herring oil and squid liver oil, and butter oil, beef tallow, and lard. Among them, corn oil, soybean oil, palm oil and coconut oil are preferable in connection with the stability of oil. Additionally, butter oil is also preferable in tight of the flavor. These oils and fats may be used singly or in mixture of two or more thereof.

Alternatively, fatty acids for use in ester exchange and ester synthetic reactions specifically include, but are not limited to, free conjugated linoleic acid, eleostearic acid and parinaric acid and the like. Additionally, extracts from natural origin and at high contents thereof, as well as fatty adds with plural conjugated dienes as recovered by synthetic processes, may also be used. Among these fatty acids, conjugated linoleic acid (CLA 80 Active Linol, Tonalin and the like as commercially available products) is preferable, because the various physiological effects have been reported.

In case of ester exchange reaction, the ratio of fats and oils as the base and conjugated fatty acid is important in order that the content of conjugated fatty acid in the triglyceride can be 20 wt% or more. The ratio in weight of fats and oils to conjugated fatty acid is preferably serected to 3.5 or less, more preferably 3 or less. Although the reaction progresses even by the addition of organic solvents, the reaction sufficiently progresses at a temperature where the mixture of fats and oils with conjugated fatty acid can be retained at a solution state, even in the absence of any organic solvent

Any lipases catalyzing ester exchange and ester synthetic reactions can be utilized for the purpose of the present invention. Commercially available examples thereof include Pancreatin F, Lipases A, F, M, AY, G, P, S and Newlase F (Trademarks, Amano Pharmaceutical Co., Ltd.; current corporate name is Amano Enzyme, Inc.); Lipase CV (Trademark, Asahi Kasei Corporation); Lipases OF, AL, PL, QL (Trademarks, Meito Industry, Co., Ltd.); and Novozyme and Lipozyme (Trademarks, Novo Nordisk, Co., Ltd.; immobilized enzyme). To achieve the content of conjugated fatty acid above 20 wt%, it is preferable to use Lipases AY, P, L, CV and Novozyme and Lipozyme since they have high activities for the ester exchange and ester synthesic reactions.

The amount of such enzymes to be added is preferably within a range from 0.1 wt% to 15 wt%, more preferably 2 wt% to 7 wt%, to the amount of the substrates in case that the enzymes are immobilized enzymes. When Lipases AY, P, L, CV are used, alternatively, the amount of the enzymes to be added is preferably within a range from 0.1 wt% to 10 wt%, more preferably 0.5 wt% to 3 wt%, to the amount of the substrates.

Additionally, to adjust the content of conjugated fatty acid in the glyceride derivative above 20 wt%, the conditions for the ester exchange reaction or ester synthesic reaction should be optimized. Otherwise, it is difficult to adjust the content. Specifically, reaction conditions such as selected enzymes, ratio between fats and oils and fatty acids functioning as substrates and water content adjustment are important.

Ester exchange reaction hardly progresses without water. It is prefable for the maximum reaction progress to adjust the water content in the reaction system within the range from 150 ppm to 2,000 ppm. Since no water generates during the ester exchange reaction, the water content in the reaction system is preferably adjusted prior to the initiation of the reaction, so as to attain a sufficient content of conjugated fatty acid, for example 20 wt% or more.

During ester synthetic reaction, alternatively, water secondarily generated is preferably dehydrated by means of pressure reduction and/or heating of the reaction system, addition of molecular sieve, drying of the circulating gas phase and the like. The resulting reaction product can almost reflect the conjugated fatty acid composition used as the substrate.

In case that unreactive fatty acid or glycerol remains at an extent to affect the quality after the termination of the ester synthesis or exchange reaction, separation and removal thereof are sometimes needed. The reaction product is frequently a mixture of mono-, di- and triglycerides, and therefore, it is sometimes required to fractionate them. To remove those unnecessary components from the reaction product, molecular distillation, water rinsing, saponification and fractionation, chromatography and the like are suggested. Industrially, molecular distillation is the most frequently used, with the resultant great fractionation level. The method for saponification and fractionation includes a method comprising saponifying fatty acid and fractionating the resulting soap into an aqueous solvent, which is then discarded, and a method comprising discarding the soap in the form of insoluble salt or the like. It is difficult to effect these methods unless the residual fatty acid is below 20 wt% in these cases, because of the high viscosity. Chromatography yields a great fractionation level but is costly.

Further, the conjugated fatty acid glyceride may be produced by allowing a microorganism with a potency to generate conjugated double bonds or an enzyme generated by the microorganism, to react with a fatty acid glyceride containing at least one of fatty acids with two or more double bonds as the constitution component or a fat/oil containing material which contains the fatty acid glyceride. Herein, the potency to generate conjugated double bonds means a potency to directly convert the fatty acid glyceride containing fatty acids with two or more double bonds as the constitution component or a fat/oil containing material which contains the fatty acid glyceride, into conjugated fatty acid glyceride.

The fatty acid glyceride containing at least one of fatty acids with two or more double bonds as the constitution component includes glycerides with one or more of fatty acids with two or more double bonds as the constitution components, for example linoleic add, α-linolenic acid, γ-linolenic add, arachidonic add, eicosapentaenoic acid (EPA), docosahexaenoic acid (DHA) and the like.

The above-mentioned fat/oil containing material at an enriched content of these glycerides specifically includes vegetable oils such as safflower oil, soybean oil, corn oil, rapeseed oil, cotton seed oil, sunflower oil, safflower oil, sesame seed oil, olive oil, linseed oil and perilla oil; and animal oils such as butter oil, fish oil, lard, and beef tallow. More specifically, vegetable oils for example safflower oil, soybean oil, corn oil, rapeseed oil, cotton seed oil, sunflower oil and safflower oil are preferable, because these oils are at large contents of fatty acids with 18 carbon atoms, particularly linoleic acid.

The microorganism with a potency to generate conjugated double bonds in accordance with the invention means a microorganism with a potency to directly convert the glyceride or the raw fat/oil containing material as the reaction starting material as described above into conjugated fatty acid glyceride. During the reaction, the microorganism may be subjected to the reaction as it is or the microorganism may be satisfactorily used as a bioreactor, after the microorganism is immobilized on a carrier. Additionally, the enzyme generated by the microorganism is recovered, purified, or pulverized, which is then used as a bioreactor as it is or after immobilization on an appropriate carrier.

Any microorganism with a potency to generate conjugated double bonds may be satisfactorily used in accordance with the invention, with no limitation. The microorganism with a potency to generate conjugated double bonds in accordance with the invention includes for example many of microorganisms for traditional use for fermented dairy products. From the respect of safety profiles, such as no pathogenicity as certified, the microorganism preferably includes intestinal microorganisms, namely lactic acid bacteria of genera *Lactobacillus, Lactococcus, Streptococcus, Enterococcus* and *Leuconostoc,* and bacteria of genera *Bifidobacterium, Eubacterium,* and *Propionibacterium.*

As to the microorganism with a potency to generate conjugated double bonds, specifically, the bacteria of the genus *Lactobacillus* are selected from for example *Lactobacillus acidophilus, Lactobacillus brevis, Lactobacillus buchneri, Lactobacillus casei, Lactobacillus delbrueckii* (ss. *delbrueckii*), *Lactobacillus delbrueckii* (ss. *lactis*), *Lactobacillus delbrueckii* (ss. *bulgaricus*), *Lactobacillus gasseri*, *Lactobacillus helveticus, Lactobacillus johnsonii*, *Lactobacillus oris*, *Lactobacillus reuteri, Lactobacillus rhamnosus, Lactobacillus zeae* and *Lactobacillus sakei.*

The bacteria of the genus *Lactococcus* are selected from for example *Lactococcus lactis, Lactococcus plantarum,* and *Lactococcus raffinolactis*. The bacteria of the genus *Leuconostoc* are selected from for example *Leuconostoc lactis.* The bacteria of the genus *Streptococcus* are selected from for example *Streptococcus thermophilus.* The bacteria of the genus *Enterococcus* are selected from for example *Enterococcus faecalis* and Enterococcus *faecium.* The bacteria of the genus *Bifidobacterium* are selected from for example *Bifidobacterium adolescentis, Bifidobacterium bifidum, Bifidobacterium breve, Bifidobacterium catenulatum, Bifidobacterium infantis* and *Bifidobacterium longum.* The bacteria of the genus *Eubacterium* are selected from for example *Eubacterium aerofaciens, Eubacterium biforme* and *Eubacterium coprostanoligens.* The bacteria of the genus *Propionibacterium* are selected from for example *Propionibacterium freudenreichii.*

Among them, preference is given to *Lactobacillus acidophilus, Lactobacillus brevis, Lactobacillus delbrueckii* (ss. *delbrueckii*), *Lactobacillus delbrueckii* (ss. *Bulgaricus*), *Lactobacillus gasseri, Lactobacillus helveticus, Lactobacillus johnsonii, Lactobacillus oris, Lactobacillus reuteri, Lactobacillus sakei, Bifidobacterium bifidum, Bifidobacterium infantis, Eubacterium biforme, Eubacterium coprostanoligens,* and *Propionibacterium freudenreichii,* because of their high generation potencies of conjugated fatty acid glyceride.

More specifically, the microorganism includes for example *Bifidobacterium bifidum* strain YIT4007 (FERM BP-791); *Bifidobacterium infantis* strains YIT4018 (ATCC15697) and YIT4019 (ATCC15702); *Lactobacillus acidophilus* strain YIT0070 (ATCC4356); *Lactobacillus brevis* strains YIT0033 (NIRD T-6) and YIT0076 (ATCC14869); *Lactobacillus delbrueckii* (ss. *delbrueckii*) strain YIT0080 (ATCC9649); *Lactobacillus delbrueckii* (ss. *bulgaricus*) strain Y1T0181 (ATCC11842); *Lactobacillus gasseri* strains YIT0168 and YIT0192 (DSM20243); *Lactobacillus helveticus* strains YIT0083 (ATCC15009) and YIT0085 (ATCC521); *Lactobacillus johnsonii* strain YIT0202 (JCM1022); *Lactobacillus oris* strain YIT0277(NCFB2160); *Lactobacillus reuteri* strain YIT0313; *Lactobacillus sakei* strain YIT0247 (JCM1157); *Propionibacterium freudenreichii* strain ATCC6207; *Eubacterium biforme* strain YIT6076 (ATCC27803); and *Eubacterium coprostanoligens* strain YIT6166 (ATCC51222).

For the method using such microorganisms in the case that the microorganisms are used as they are, rinsed fresh bacterial cells are mixed with emulsified oils and fats for reaction, or the microorganisms are inoculated and cultured in a food containing fat and oil materials, or rinsed fresh bacterial cells are mixed with a food containing fat and oil materials, for reaction. The resulting products are satisfactorily used for the method. When immobilizing the microorganisms for use as bioreactor, for example, bacterial cell-derived enzymes are used as they are. Otherwise, fresh bacterial cells are ground, for enzyme purification by general fractionation processes such as salting-out and film process, ion exchange resin process, and gel filtration process, followed by immobilization of the resulting enzyme fraction onto silica gel, Celite, ion exchange resins, chitosan beads and cellulose through ion bonding-, covalent bonding- and hydrophobic bonding methods.

The resulting reaction product is used, as it is or preferably after additional purification. Specifically, the purification method comprises recovering an oil phase by centrifugation in case that the reaction product is suspended in an aqueous phase, and extracting the reaction product in an organic solvent for purification. As the extraction solvent, solvents capable of solubilizing conjugated fatty acid glyceride with conjugated double bonds are used according to general methods. As the reaction composition containing the thus recovered conjugated fatty acid glyceride, the fractionated oil phase can be used as it is.

For the conjugated fatty acid glyceride of the present invention, any fatty acid with conjugated double bonds within the molecule specifically including conjugated linoleic acid, eleostearic acid and parinaric acid, can preferably be used after modification into glyceride forms. Additionally, the glyceride forms may satisfactorily be any of mono-, di- or triglycerides. However, conjugated fatty acid glyceride at a higher content of di- and triglycerides containing more conjugated fatty add within one molecule is preferred.

Compared with not only free fatty acid but also other esters such as methyl ester, the conjugated fatty acid modified into such glyceride forms can have higher effects on the improvement of taste and the promotion of digestion and absorption, and also can have enhanced effects on anti-obesity, the improvement of lipid metabolism and the prophylaxis and therapeutic treatment of hyperlipidemia, owing to the higher effects described above.

So as to incorporate conjugated fatty acid glyceride at an effective amount promising the physiological effects (1 to 3 g daily in case of conjugated linoleic acid, for example) within an appropriate fat intake range, the content of conjugated fatty acid in the derivative is preferably 20 % or more. Below 20 % of the conjugated fatty acid content in the glyceride, 5 to 15 g of the derivative is to be incorporated, which leads to the increase of calorie intake, so that the possible physiological effects of conjugated fatty acid, for example the decrease of body fat and the prophylactic effect of cancer, are potentially reduced.

The conjugated fatty acid glyceride thus recovered absolutely never causes safety concerns. Additionally, the ingestion of such conjugated fatty acid glyceride is preferably 100 mg to 5,000 mg, particularly 300 mg to 3,000 mg daily for each person.

The conjugated fatty acid glyceride recovered by the various reactions in accordance with the present invention may be used as it is in the fat and oil composition containing the conjugated fatty acid glyceride. The conjugated fatty acid glyceride may be mixed with and processed into common foods and drinks or tablets, capsules, granules and salad oil-like foods, for use. Owing to the excellent digestion and absorption properties of the conjugated fatty acid glyceride, these formulations, mainly capsules and granules, are preferable, particularly because the formulations can overcome the disadvantages of free-form conjugated fatty acid glyceride, such as the reduction of absorption efficiency, depending on the fasting degree during intake.

More specifically, the fat and oil composition containing the conjugated fatty acid glyceride recovered through various reactions in accordance with the present invention can be processed by for example fractionation, sterilization, mixing, concentration and drying, which can then be used in combination with other foods and drinks and other substances for oral administration, as medical products or foods and drinks. After mixed with various flavoring materials, juice powders, sweeteners such as sugar and fructose, sour agents such as citric acid and malic acid, stabilizers, thickeners, cereal powders, vitamins and minerals, the composition can be used in the forms of bread, noodles, pastas, baked confectionaries, cream, candies, chewing gum, tablet sweets, tea, coffee, juice drinks, fermented milk, carbonate drinks, pudding and jellies. Similarly, the composition may be processed into tablets, capsules, granules or salad oil-like foods for use in the form of nutritional or dietary supplements.

In case of using the conjugated fatty acid glyceride in the form of foods in combination with lipid other than conjugated fatty acid, preferably, fat and oil materials with smaller linoleic acid contents are used. Specifically, use is preferably made of oils and fats such as butter oil, fish oil, perilla oil, perilla oil, palm oil, coconut oil, linseed oil, olive oil, beef tallow and lard. When fats and oils at larger contents of linoleic acid are used in combination, the physiological effects of conjugated fatty acid, namely the anti-obesity effect, the effect on the improvement of the metabolism of fats and oils, the prophylactic and therapeutic effect of hyperlipidemia, may potentially be suppressed.

In the conjugated fatty acid glyceride of the invention, the bitterness and astringency specific to conjugated fatty acid are suppressed. Additionally, the digestion and absorption properties of conjugated fatty acid are also improved. Thus, the use of the conjugated fatty acid glyceride can promise excellent taste and efficient absorption, whenever the glyceride is ingested in any circumstance.

The use of the conjugated fatty acid glyceride for improving lipid metabolism in accordance with the present invention additionally enables continuous intake of conjugated fatty acid with various great physiological effects, with no need of combined use of various taste-modifying materials, with the resulting great effects of organ fat reduction, anti-obesity, and body weight reduction, as well as possible effects on anti-diabetes mellitus, anti-arteriosclerosis, and anti-cancer. Additionally, the conjugated fatty acid glyceride containing conjugated linoleic acid within the molecule can prominently improve hyperlipidemia and reduce the risks of arteriosclerosis, diabetes mellitus and cardiovascular diseases and the like.

### BRIEF DESCRIPTION OF THE DRAWINGS

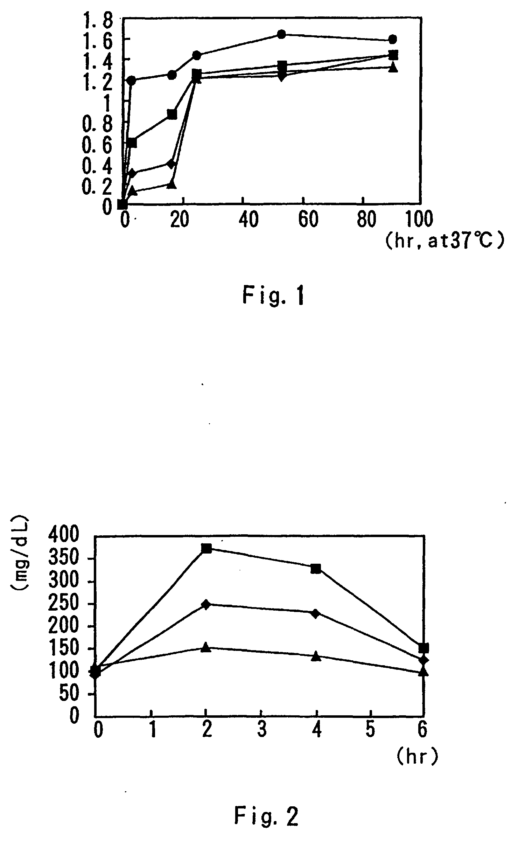

Fig. 1 shows graphs of the change of the value of conjugated linoleic acid in the glyceride during ester exchange reaction between various fat and oil materials and conjugated linoleic acid over time; where the ordinate denotes the value of conjugated linoleic acid in the glyceride, while the abscissa denotes the reaction time (hr) at 37 °C; and ◆ shows butter; ■, corn oil; ▲, coconut oil; and ●, sardine oil.

Fig. 2 shows graphs of the absorption velocity of conjugated linoleic acid-containing lipid; where the ordinate denotes the blood triglyceride concentration (mg/dL), while the abscissa denotes the time (hr) after administration of the lipid through a stomach probe to ICR mice; and ◆ shows CLA (conjugated linoleic acid); ■, CLA-TG (conjugated linoleic acid-containing triglyceride); and ▲, control.

### DESCRIPTION OF THE PREFERRED EMBODIMENTS

The present invention will now be described in more detail in the following embodiments, but the present invention is not limited to these embodiments.

### Example 1: Conjugated Linoleic Acid Glyceride Production 1; Ester Exchange Reaction:

In a 300-mL medium bottle were placed an enzyme Lipase PL (1 g), 400 mL of water, 95 mL of hexane, and 58 g each of butter oil, coconut oil or corn oil or 69 g of sardine oil, followed by addition of Active Linol (product name; manufactured by RINORU OIL MILLS, Co., Ltd.; containing conjugated linoleic acid at 73.6 %), and then, nitrogen was filled in the bottle for sealing. The resulting mixture reacted together under agitation with a reciprocal agitator at 37 °C for 91 hours. At a predetermined interval, a sample of 10 mL was collected and diluted, for thin layer chromatography analysis. The results are shown in Fig. 1. Because the fatty acid in the fats and oils and the conjugated linoleic acid were set at equivalent moles for the reaction, the value of the conjugated linoleic acid in the glyceride when the reaction reached equilibrium was calculated as 1.5. It is indicated that the content of conjugated linoleic acid incorporated in the glyceride exceeded 20 % (corresponding to 0.6 value of conjugated linoleic acid in the glyceride) in two hours after the start of the reaction in case of butter oil and sardine oil.

### Example 2: Conjugated Linoleic Acid Glyceride Production 2; Ester Synthesis Reaction:

100 g of CLA 80 (product name; manufactured by RINORU OIL MILLS, Co., Ltd.), 11g of glycerol (for food additives) and 11.3 g of Lipozyme IM (product name; manufactured by Novo Nordisk, Co., Ltd.) are weighed in a round bottom flask. While the mixture was dried and solidified under reduced pressure in a rotary evaporator, the mixture was agitated at about 70 °C, for ester synthesis reaction for 12 hours. Lipozyme IM was filtered and recovered from the reaction product, to recover conjugated linoleic acid glyceride. The resulting reaction product was determined by thin layer chromatography and iodine color reaction. Consequently, triglyceride was at 85.5 %; diglyceride, at 13.5 %; monoglyceride, at 1 % or less; unreactive fatty acid, at 4 %; and unreactive glycerol, at 2 % or less.

### Example 3: Screening of Lactic Acid Bacteria:

Bacteria with a potency reacting with fats and oils containing linoleic acid to directly generate conjugated linoleic acid were screened. First, various lactic acid bacteria shown in Tables 1 and 2, bacteria of the genus *Bifidobacterium* and bacteria of the genus *Propionibacterium* were cultured in 0.035 % linoleic acid-containing MRS culture medium; the bacterial cells were harvested by centrifugation and rinsed in physiological saline to prepare rinsed bacterial cell. Further, the bacteria of the genus *Eurobacterium* were treated in the same manner as described above, except for cultivation under anaerobic conditions using a BCM culture medium as the culture medium therefor.

These rinsed fresh bacterial cells of 100 mL were mixed in 1 mL of 50 mM phosphate buffer, pH 7.1 containing 5 mg of linoleic acid, for shaking at 37 °C for 2 days, for reaction. After subsequent centrifugation and extraction of the supernatant in the 2-fold volume of chloroform: methanol (1 : 2), lipid was extracted in the chloroform layer. The extracted lipid was appropriately diluted with hexane, for ultraviolet absorptiometry, to assay the generated conjugated linoleic acid.

Still further, bacterial strains which were verified of their conjugated linoleic acid generation were treated by the same method, using an emulsion containing 5 mg of corn oil instead of linoleic acid, for ultraviolet absorptiometry. Herein, the linoleic acid content in corn oil is about 50 %.

Consequently, it was shown that the bacteria marked with circle in Tables 1 and 2 have a potency to convert the linoleic acid glyceride-containing fats and oils into conjugated linoleic add glyceride. Additionally, the presence or absence of free fatty acid in the extracted lipid was assayed by thin layer chromatography. Almost no band of free fatty acid was detected. Hence, it was shown that the generated conjugated fatty acid was of glyceride type.

Because it is demonstrated that some bacteria have a poor potency to isomerize glyceride-type linoleic acid although the bacteria can strongly isomerize linoleic acid (bacteria marked with "× " in the column corn oil in each Table), it is indicated that bacteria isomerizing linoleic add cannot essentially isomerize glyceride-type linoleic acid.

**Table 1**

| No | Genus | Species | Potency * | | YIT No. | Accession No. |
|---|---|---|---|---|---|---|
| | | | L.A.** | C.O.*** | | |
| 1 | Bifidobacterium | adolescentis | × | | YIT4011 | ATCC15703 |
| 2 | Bifidobacterium | adolescentis | × | | YIT4087 | JCM7042 |
| 3 | Bifidobacterium | bifidum | ○ | ○ | YIT4007 | FERM BP-791 |
| 4 | Bifidobacterium | breve | ○ | × | YIT4014 | ATOC15700 |
| 5 | Bifidobacterium | breve | × | | YIT4065 | |
| 6 | Bifidobacterium | catenulatum | × | | YIT4016 | ATOC27539 |
| 7 | Bifidobacterium | catenulatum | ○ | × | YIT4118 | JCM7130 |
| 8 | Bifidobacterium | infantis | ○ | ○ | YIT4018 | ATOC15697 |
| 9 | Bifidobacterium | infantis | ○ | ○ | YIT4019 | ATOC15702 |
| 10 | Bifidobacterium | lactis | ○ | × | YIT4121 | DSM10140 |
| 11 | Bifidobacterium | longum | ○ | × | YIT4021 | ATOC15707 |
| 12 | Bifidobacterium | longum | × | | YIT4037 | ATOC15708 |
| 13 | Bifidobacterium | pseudocatenulatum | × | | Y1T4072 | JCM1200 |
| 14 | Lactobacillus | acidophilus | ○ | ○ | YIT0070 | ATOC4356 |
| 15 | Lactobacillus | brevis | ○ | ○ | YIT0033 | NIRD T-6 |
| 16 | Lactobacillus | brevis | ○ | ○ | YIT0076 | ATOC14869 |
| 17 | Lactobacillus | buchneri | × | | YIT0040 | NIRD BC-1 |
| 18 | Lactobacillus | buchneri | × | | YIT0077 | ATOC4005 |
| 19 | Lactobacillus | casei | × | | YIT0180 | ATOC334 |
| 20 | Lactobacillus | casei | × | | YIT9018 | |
| 21 | Lactobacillus | casei | × | | YIT9029 | |
| 22 | Lactobacillus | delbrueckii(ss.delbrueckii) | ○ | ○ | YIT0080 | ATOC9649 |
| 23 | Lactobacillus | delbrueckii(ss.lactis) | × | | YIT0058 | NIRD L-1 |
| 24 | Lactobacillus | delbrueckii(ss.lactis) | × | | Y1T0086 | ATOC12315 |
| 25 | Lactobacillus | delbrueckii(ss.bulgaricus) | ○ | ○ | YIT0181 | ATOC11842 |
| 26 | Lactobacillus | gasseri | ○ | ○ | YIT0168 | |
| 27 | Lactobacillus | gasseri | ○ | ○ | YIT0192 | DSM20243 |
| 28 | Lactobacillus | helveticus | ○ | ○ | YIT0083 | ATOC15009 |
| 29 | Lactobacillus | helveticus | ○ | ○ | YIT0085 | ATOC521 |
| 30 | Lactobacillus | johnsonii | ○ | ○ | YIT0202 | JCM1022 |

| | | | | | | |
|---|---|---|---|---|---|---|
| * : Conjugated linoleic acid glyceride generation potency | | | | | | |
| ** : Linoleic acid | | | | | | |
| ***: Corn oil | | | | | | |

**Table 2**

| No | Genus | Species | Potency * | | YIT No. | Accession No. |
|---|---|---|---|---|---|---|
| | | | L.A.** | C.O.*** | | |
| 31 | Lactobacillus | oris | ○ | ○ | YIT0277 | NCFB2160 |
| 32 | Lactobacillus | parabuchneri | × | | YIT0272 | NRIC1780 |
| 33 | Lactobacillus | pentosus | × | | YIT0238 | JCM1558 |
| 34 | Lactobacillus | plantarum | × | | YIT0101 | ATOC8014 |
| 35 | Lactobacillus | plantarum | × | | YIT0102 | ATOC14917 |
| 36 | Lactobacillus | pontis | × | | YIT0273 | DSM8475 |
| 37 | Lactobacillus | reuteri | ○ | ○ | YIT0313 | |
| 38 | Lactobacillus | rhamnosus | ○ | × | YIT0105 | ATOC7469 |
| 39 | Lactobacillus | rhamnosus | ○ | × | YIT0124 | ATOC9595 |
| 40 | Lactobacillus | salivarius(ss.salicinius) | × | | YIT0089 | ATOC11742 |
| 41 | Lactobacillus | salivarius(ss.salivarius) | × | | YIT0104 | ATOC11741 |
| 42 | Lactobacillus | sanfrancisco | × | | YIT0240 | JCM5668 |
| 43 | Lactobacillus | sakei(ss.sakei) | ○ | ○ | YIT0247 | JCM1157 |
| 44 | Lactococcus | ctis(ss.cremoris) | × | | YIT2002 | ATOC14365 |
| 45 | Lactococcus | ctis(ss.cremoris) | × | | YIT2007 | ATOC19257 |
| 46 | Lactococcus | ctis(ss.lactis) | × | | YIT2008 | ATOC19435 |
| 47 | Lactococcus | ctis(ss.lactis) | × | | YIT2027 | |
| 48 | Lactococcus | antarum | × | | YIT2061 | ATOC43199 |
| 49 | Lactococcus | ffinolactis | × | | YIT2062 | ATOC43920 |
| 50 | Leuconostoc | lactis | ○ | × | YIT3001 | ATOC19256 |
| 51 | Leuconostoc | lactis | × | | YIT3004 | NCD0532 |
| 52 | Streptococcus | thermophilus | × | | YIT2001 | |
| 53 | Streptococcus | thermophilus | × | | YIT2037 | ATOC19258 |
| 54 | Propionibecterium | freudenreichii | ○ | ○ | | ATOC6207 |
| 55 | Eubacterium | rofaciens | × | | Y 98010 | ATOC25986 |
| 56 | Eubacterium | mosum | × | | YIT6067 | JCM6421 |
| 57 | Eubacterium | forme | ○ | ○ | YIT6076 | ATOC27806 |
| 58 | Eubacterium | ntum | × | | YIT6077 | ATOC25559 |
| 59 | Eubacterium | ctale | × | | YIT6082 | ATOC33656 |
| 60 | Eubacterium | prostanoligens | ○ | ○ | YIT6166 | ATOC51222 |

| | | | | | | |
|---|---|---|---|---|---|---|
| * : Conjugated linoleic acid glyceride generation potency | | | | | | |
| ** : Linoleic acid | | | | | | |
| ***: Corn oil | | | | | | |

### Example 4: Preparation and Absorption Test of Glyceride:

The reaction product of 9 g (the quantity of residual fatty acid was 3.6 g; the quantity of glyceride was 5.4 g; the conjugated linoleic acid content in glyceride was at 35 %) as recovered in Example 1 with corn oil was preliminarily equilibrated with a buffer, pH 7, and was then absorbed onto a DEAE cellulose column (dry resin quantity of 60 g; column with a diameter of 3.5 cm and 30 cm in length) substituted with ethanol and then with hexane, followed by elution with hexane. Then, the non-adsorbed fraction was recovered, from which the solvent was removed with a rotary evaporator, to recover a dry solid product of 5 g. The lipid composition of the product was assayed by thin layer chromatography. Consequently, the product was at a composition of 96 % of glyceride derivative, with 4 % of contaminated fatty acid, so conjugated linoleic acid occupied 29 % of the dry solid product.

The dry solid product was administered through a stomach probe to male ICR mice of age 19 weeks after overnight fasting (5 mL/kg; n = 5). As a control, a mixture of 3 parts by weight of Active Linol (manufactured by RINORU OIL MILLS, Co., Ltd.; containing 70 % of conjugated linoleic acid) and 4 parts of linoleic acid was similarly administered. As an additional control, a group with no administration was prepared. Over time, blood was taken from orbit in the 3 groups, to assay blood triglyceride. Consequently, it was shown that the glycerol derivative of the invention had greater digestion and absorption properties than those of the control free fatty acid type (Fig. 2). Blood samples taken from two animals in each of the groups were assayed for fatty acid analysis by gas chromatography. The presence of conjugated linoleic acid was confirmed in any of the blood samples.

### Example 5: Conjugated Linoleic Acid Glyceride Production 3; Preparation of Milk-based Drink containing Conjugated Linoleic Acid Glyceride:

Three bacterial strains from the lactic acid bacteria in Table 1, namely *Bifidobacterium infantis* strain YIT4018, *Lactobacillus delbrueckii* ss. *bulgaricus* strain YIT0181 and *Lactobacillus helveticus* strain YIT0085, were tested about conjugated linoleic acid glyceride generation. While dropwise adding safflower oil (containing linoleic acid at 73 %) to an equal volume of an aqueous solution containing polyglycerin fatty add ester (MSW750, trade name; Sakamoto Yakuhin Kogyo, Co., Ltd .) at 0.5 % to the weight of the fats and oils, the safflower oil was emulsified with an emulsifier Physcotron to prepare an emulsion of fats and oils at 50 %. The bacterial cells of the individual bacterial strains cultured in 0.035 % linoleic acid-containing MRS culture medium were rinsed. To the emulsion were added the resulting fresh rinsed bacterial cells of a weight in part by weight of about 1 / 20-fold the total weight of the fats and oils on a wet weight basis, for reaction at 37 °C for 2 days. From the reaction solution was extracted fat, which was appropriately diluted with hexane for ultraviolet absorptiometry, to examine the generation of conjugated linoleic acid glyceride. As in Example 3, the quantity of free fatty acid was assayed by thin layer chromatography.

Consequently, it was shown that conjugated linoleic acid glyceride was generated in all of the *Bifidobacterium infantis* strain YIT4018, *Lactobacillus delbrueckii* ss. *bulgaricus* strain YIT0181, and *Lactobacillus helveticus* strain YIT0085.

Additionally, fats and oils containing conjugated linoleic acid as prepared in the Lactobacillus helveticus strain YIT0085 were added to and mixed with milk products, to produce a milk-based drink containing conjugated linoleic acid glyceride. Specifically, 0.4 part by weight of the fats and oils was emulsified in and mixed with 99.6 parts by weight of low-fat milk adjusted to a 1.1 % milk fat content by partially removing cream with a centrifuge, to produce a milk-based drink containing conjugated linoleic acid glyceride. This milk-based drink was highly tasty and had stable physico-chemical properties.

### Example 6: Conjugated Linoleic Acid Glyceride Production 4; Preparation of Soybean Milk Drink containing the same:

Among the lactic acid bacteria shown in Tables 1 and 2, three bacterial strains, namely *Bifidobacterium bididum* strain YIT4007, *Lactobacillus helveticus* strain YIT0085 and *Lactobacillus reuteri* strain YIT0313, were tested about their generation of conjugated linoleic acid glyceride during the production of fermented soybean milk.

Soybean milk (at no-fat solid content of 8.3 %) containing soybean oil at 5 % (containing linoleic acid at 52 % and linolenic acid at 8 %) was sterilized under heating at 100 °C for 60 minutes, to which were inoculated the individual bacterial strains, for cultivation at 37 °C for 2 days, to produce fermented soybean milks. Fat was extracted from the individual fermented soybean milks, to assay the absorbance at 235 nm and examine the generation of conjugated linoleic acid.

Consequently, it was shown that all the bacterial strains converted linoleic acid to conjugated linoleic acid. Although a possibility remained that a part of the conjugated double bonds might be derived from linolenic acid, the possibility could not be verified. Additionally because free fatty acid was not increased in the fermented soybean milks, it was shown that the generated conjugated linoleic acid was derived from the conversion of the linoleic acid per se in the glyceride derivative into conjugated linoleic acid.

### Example 7: Preparation of Bacterial Enzyme:

The 10-mL culture of the *Lactobacillus helveticus* strain YIT0085 among the lactic add bacteria from dairy origin as shown in Table 1 was rinsed to recover fresh bacterial cells, which were then suspended in 2 mL of a 10 % lysophosphatidyl glycerol solution, for treatment under heating at 50 °C for 30 minutes, to prepare the bacterial enzyme. According to the method in Example 5, the enzyme was added in place of the fresh bacterial cells, for reaction. It was confirmed by ultraviolet absorptiometry and thin layer chromatography that conjugated fatty acid glycerides were generated.

### Example 8: Anti-obesity Test:

The conjugated linoleic acid-containing glyceride (TG-CLA) recovered in Example 2 was given at 0.5 % to mice from which the ovaries were preliminarily resected, to examine the influence thereof on organ fat deposition. Specifically, thirty six (36) C57BL/6J mice (female) of age 6 weeks were purchased from Nippon SLC, Co., Ltd. After preliminary feeding the mice with MF diet for one week, left and right ovaries were resected from the mice according to general procedures.

After 1-week recovery, the mice were divided in 4 groups , each group consisting of 9 mice. The experimental feeds A to D shown in Table 3 were individually fed ad libitum for 7 weeks. After overnight fasting, the mice were subjected to laparotomy under anesthesia with ether. Immediately after blood collection, fat tissue around kidney, uterus and mesentrium was resected and weighed, which was designated organ fat weight Throughout the experimental duration, the feeding conditions were at constant temperature (24 ± 1 °C) and constant humidity (60 ± 5 %), while the bright and dark cycle was set to 12 hours. The feeds and water were fed ad libitum. The body weight and the feed intake were measured every week.

Concerning the feed intake, herein, feed intake of 3 mice per one week was measured, because mice were actually fed at a unit of 3 animals per one cage. Data were all expressed in 'mean value ± standard deviation". The difference in mean value between the individual groups was tested by the Tukey's multiple comparison.

Consequently, no difference in feed intake was observed. Additionally, the body increment (%) during the experimental term was 35.8 ± 5.4 % in the inventive group A, 38.3 ± 8.8 % in the corn oil group B, 47.5 ± 11.3 % in the linseed oil group C or 40.2 ± 5.9 % in the fish oil group D. Compared with the other groups, the linseed oil group C was at a larger body weight increment, and on weeks 6 and 7, the body weight increment in the inventive group A was at a significantly smaller value compared with that in the linseed oil group C (p < 0.05). Table 4 shows the body weight and organ fat weight during autopsy. The organ fat weight in the inventive group A was at a value smaller by 30 % and 38 % than those in the com oil group B and the linseed oil group C, respectively, which was significantly different (p < 0.01, p < 0.001). Although no significant difference was detected, additionally, the ratio of the organ fat weight to the body weight in the inventive group A was likely to be small (p = 0.13) compared with the fish oil group D which is said to reduce body fat. Thus, excellent effects of the inventive product could be confirmed.

**Table 3 :**

| COMPOSITION OF EXPERIMENTAL FEED (wt%) | | | | |
|---|---|---|---|---|
| | Feed A | Feed B | Feed C | Feed D |
| Casein | 24 | 24 | 24 | 24 |
| Soybean peptide ^{*)} | 1 | 1 | 1 | 1 |
| Corn starch | 13.3 | 13.3 | 13.3 | 13.3 |
| Sucrose | 50 | 50 | 50 | 50 |
| Com oil | 4.5 | 5 | - | - |
| TC-CLA of Example 2 | 0.5 | - | - | - |
| Linseed oil | - | - | 5 | - |
| Fish oil | - | - | - | 5 |
| Cellulose | 2 | 2 | 2 | 2 |
| Vitamin mixture ^{**)} | 1 | 1 | 1 | 1 |
| Mineral mixture ^{**)} | 3.5 | 3.5 | 3.5 | 3.5 |
| Choline hydrogen tartaric acid | 0.2 | 0.2 | 0.2 | 0.2 |

| | | | | |
|---|---|---|---|---|
| Feed A: Inventive feed containing TG-CLA of Example 2. Feed B: Comparative feed containing corn oil. Feed C: Comparative feed containing linseed oil. Feed D: Comparative feed containing fish oil. * : Hinute R (Trademark; Fuji Oil, Co., Ltd.). | | | | |
| ** : AlN 76 blended. | | | | |

**Table 4:**

| COMPARISON OF BODY WEIGHT AND ORGAN FAT WEIGHT | | | | |
|---|---|---|---|---|
| | Group A N=9 | Group B N=9 | Group C N=9 | Group D N=9 |
| Body weight (g) | 25.94 ± 1.26a | 26.80 ± 2.04 | 28.50 ± 2.10b | 28.50 ± 1.46 |
| Organ fat (g) | 1.24 ± 0.46a | 1.84 ± 0.49b | 2.17 ± 0.41d | 1.63 ± 0.25 |
| Organ fat (%) | 4.97 ± 1.51a | 7.12 ± 1.40c | 7.97 ± 0.97d | 6.31 ± 0.78 |
| A vs b, p < 0.05; a vs c, p < 0.01; a vs d, p < 0.001 (Tukey's multiple comparison test) Group A: Group fed with Feed A containing TG-CLA of Example 2. Group B: Group fed with Feed B containing corn oil. Group C: Group fed with Feed C containing linseed oil. Group D: Group fed with Feed D containing fish oil. | | | | |

### Example 9: Blood Triglyceride Comparative Test 1:

From C57BL/6J mice of age 7 weeks were resected left and right ovaries. The resulting mice were divided in 3 groups, which were individually fed ad libitum with MF feed supplemented with 1.2 % safflower oil (control group), MF feed containing 0.67% safflower oil and the conjugated linoleic acid glyceride prepared by the same method as in Example 2 (TG-CLA) at 0.53 % (0.53 % TG-CLA group), and MF feed supplemented with 1.2 % TG-CLA (1.2 % TG-CLA group). As shown in Table 5, the fatty acid composition of the TG-CLA used was mainly occupied with cis-9, trans-11/trans-9, cis-11,18:2 and trans-10, cis-12, 18:2, and was at a triglyceride content of 80 % or more.

After 8-week feeding and 4-hour fasting, blood was taken from the hearts of the 8 mice of each group under ether anesthesia, to assay blood triglyceride, by using a commercially available kit (Triglyceride Test Wako). The results are shown in Table 6. Herein, blood neutral fat was at a significantly small value in the 1.2 % TG-CLA group.

**Table 5 :**

| Composition of TG-CLA | |
|---|---|
| Fatty add composition | (%) |
| 16:0 | 6.9 |
| 18:0 | 2.7 |
| 18:1 | 15.1 |
| 18:2 (non-conjugated) | 0.8 |
| Conjugated linoleic acid 18:2 | 73.6 |
| (c9, t11 / t9, c12) | (34.5) |
| (t10, c12) | (35.2) |
| (other isomers) | (3.9) |

| Glyceride derivative composition | (%) |
|---|---|
| Triglyceride | 83.4 |
| Diglyceride | 12.4 |
| Monoglyceride | 0 |
| Free fatty acid | 4.2 |

**Table 6 :**

| Blood triglyceride comparison | | |
|---|---|---|
| | Blood TG (mg/dL) | p |
| Control group | 42.0 ± 8.5 | |
| 0.53 % TG-CLA group | 39.0 ± 6.8 | 0.590 |
| 1.2 % TCrCLA group | 29.0 ± 4.7 | 0.002 |
| p : Dunnet's multiple comparison test; 8 mice per group | | |

As shown in Table 6, it was indicated that the substitution of a part of lipid in diets with conjugated linoleic acid glyceride could allow the effect of reducing blood neutral fat to be exerted sufficiently. This indicates that hyperlipidemia caused by menopausal metabolic abnormalities can effectively be reduced by using conjugated linoleic acid glyceride.

### Example 10: Blood Triglyceride Comparison Test 2:

After preliminary one-week feeding of male C57BL/Ksj, db/db mice of age 7 weeks, the mice were divided in 3 groups, for feeding of the same feeds as in Example 9 ad libitum. Blood was drawn out from orbit, prior to the dosing and on weeks 2, 4, 6 after the dosing, to assay blood neutral fat. The results are shown in Table 7. Compared with the control group, further, blood triglyceride was at significantly small values in the test groups.

As shown in Table 7, it was indicated like Example 9 that the substitution of a part of lipid in diets with conjugated linoleic acid glyceride could allow the effect of reducing blood neutral fat to be exerted sufficiently. This indicates that hyperlipidemia caused by Type II diabetes mellitus can be reduced effectively by using conjugated linoleic acid glyceride.

**Table 7**

| Blood triglyceride comparison | | | | |
|---|---|---|---|---|
| | Blood TG (mg/dl) | | | |
| | Prior to dosing | On week 2 | On week 4 | On week 6 |
| Control group | 373.9 ±159.5 | 768.2 ± 275.9 | 596.9 ± 151.8 | 681.4 ± 159.0 |
| 0.53 % TG-CLA group | 387.9 ± 118.5 | 448.7 ± 182.0* | 294.6 ± 75.1** | 398.8 ± 122.6** |
| 1.2 % TG-CLA group | 399.5 ± 131.8 | 485.5 ± 44.0* | 296.7 ± 99.8** | 482.7 ± 82.9* |

| | | | | |
|---|---|---|---|---|
| * : p < 0.05; | | | | |
| ** : p < 0.001 (Dunnet's multiple comparison test; 7 mice per group) | | | | |

### Example 11:Sensory Test:

Using the conjugated linoleic acid-containing glycerides of Examples 2 and 4 and a commercially available conjugated linoleic acid (Active Linol; manufactured by RINORU OIL MILLS, Co., Ltd.), 10 panelists tested the taste at sensory test Consequently, it was found that the glyceride derivatives of the invention had no bad taste derived from fatty add and that the glyceride derivatives were products with almost no taste and no odor (Table 8).

Additionally, the sensory test was performed by 10 persons, and the mean score is shown. The scoring standards are as follows: no taste with 0; slight bittemess with 1; some bitterness with 2; fair bittemess with 3; and extreme bitterness with 4.

**Table 8**

| | point |
|---|---|
| Inventive product 1 (Example 2) | 0 |
| Inventive product 2 (Example 4) | 0.1 |
| Control commercially available product (Active Linol) | 3.8 |

### Example 12: Soft Capsule Type Supplement Production 1:

According to the following prescription, a capsule base was prepared from gelatin, glycerol and water; then, an oil to be contained was encapsulated and molded with the capsule base, to prepare a soft capsule type dietary or nutritional supplement.

Prescription:

| | |
|---|---|
| gelatin | 0.4275 g |
| glycerol | 0.0225 g |
| water | 0.05 g |
| Conjugated linoleic acid glyceride of Example 2 | 2.0 g |

The resulting soft capsule type supplement had great flavor.

### Example 13: Tablet Type Supplement Production 1:

According to the following prescription, various ingredients were mixed together for tableting, to prepare a tablet type dietary or nutritional supplement

### Prescription:

| | |
|---|---|
| Lactose | 10 g |
| Conjugated fatty acid glyceride-containing oil of Example 2 | 250 mg |
| Calcium pantothenate | 10 mg |
| Vitamin B2 | 4 mg |
| DK ester F-20 W (manufactured by Dai-ichi Kogyo Seiyaku, Co., Ltd.) | 400 mg |
| Finely prepared cellulose | 6 g |
| Dextrin | 8 g |

The resulting tablet type supplement had great flavor.

### Example 14: Tablet Type Supplement Production 2:

According to the following prescription, various ingredients were mixed together for tableting, to prepare a tablet type dietary or nutritional supplement. Consequently, the resulting tablet type supplement had great flavor.

### Prescription:

| | |
|---|---|
| Lactose | 10 g |
| Conjugated fatty acid glyceride-containing oil of Example 7 | 250 mg |
| Calcium pantothenate | 10 mg |
| Vltamin B2 | 4 mg |
| DK ester F-20 W (manufactured by Dai-ichi Kogyo Seiyaku, Co., Ltd.) | 400 mg |
| Finely prepared cellulose | 6 g |
| Dextrin | 8 g |

## Claims

1. A conjugated fatty acid ester containing a conjugated fatty acid with conjugated double bonds, especially conjugated linoleic acid, within the molecule, **characterized in that** the conjugated fatty acid forms an ester bond with glycerol to form conjugated fatty acid glyceride.

2. An oral agent with an action to improve lipid metabolism, an anti-obesity action, or an action to prevent or therapeutically treat hyperlipidemia, said oral agent comprising a conjugated fatty add glyceride containing, as the effective ingredient, a conjugated fatty acid with conjugated double bonds, especially conjugated linoleic acid, within the molecule, and pharmaceutically acceptable ingredients.

3. The use of a conjugated fatty acid ester containing a conjugated fatty acid with conjugated double bonds within the molecule, in the preparation of an oral agent for use in the improvement of lipid metabolism, anti-obesity, or the prophylaxis andlor therapeutic treatment of hyperlipidemia, wherein the conjugated fatty acid within the molecule forms an ester bond with glycerol to form conjugated fatty acid glyceride.

4. The use of the conjugated fatty acid ester according to claim 3, **characterized in that** the conjugated fatty acid is conjugated linoleic acid.

5. A milk-based drink for improving lipid metabolism, comprising a milk and a conjugated fatty acid glyceride mixed with said milk, said conjugated fatty acid glyceride containing a conjugated fatty acid with conjugated double bonds within the molecule.

6. A soybean milk for improving lipid metabolism, comprising a soybean milk and a conjugated fatty acid glyceride mixed with said soybean milk, said conjugated fatty acid glyceride containing a conjugated fatty acid with conjugated double bonds within the molecule.

7. A capsule type dietary or nutritional supplement for improving lipid metabolism, comprising a capsule molded with a capsule base and a conjugated fatty acid glyceride encapsulated with the capsule, said conjugated fatty acid glyceride containing a conjugated fatty acid with conjugated double bonds within the molecule.

8. A tablet type dietary or nutritional supplement for improving lipid metabolism, comprising a tablet base and a conjugated fatty acid glyceride, said cinjugated fatty acid glyceride containing a conjugated fatty add with conjugated double bonds within the molecule.
